# EUROPÄISCHE PATENTSCHRIFT

(11) **EP 1 200 601 B1**
(45) Veröffentlichungstag und Bekanntmachung des Hinweises auf die Patenterteilung: **08.03.2006**
(21) Anmeldenummer: 00943826.8
(22) Anmeldetag: 17.06.2000
(51) Int. Cl.: C12N 15/55, C12N 11/08, C12N 9/80, C12P 41/00, C12P 13/04

(54) **VERFAHREN ZUR HERSTELLUNG VON L-AMINOSÄUREN AUS IHREN RACEMISCHEN N-ACETYL-D,L-DERIVATEN DURCH ENZYMATISCHE RACEMAT-SPALTUNG MITTELS ISOLIERTER, REKOMBINANTER ENZYME**
METHOD FOR THE PRODUCTION OF L-AMINO ACIDS FROM THEIR RACEMIC N-ACETYL-D, L-DERIVATIVES BY ENZYMATIC RACEMATE CLEAVAGE BY MEANS OF ISOLATED RECOMBINANT ENZYMES
PROCEDE DE FABRICATION D'ACIDES AMINES L A PARTIR DE LEURS DERIVES-D,L-N-ACETYLIQUES RACEMIQUES, PAR SEPARATION RACEMIQUE ENZYMATIQUE AU MOYEN D'ENZYMES RECOMBINANTES ISOLEES

(30) Priorität: 20.07.1999 DE 19933362
(43) Veröffentlichungstag der Anmeldung: 02.05.2002
(73) Patentinhaber: Bayer CropScience GmbH, 65929 Frankfurt/Main (DE)
(72) Erfinder: BARTSCH, Klaus, D-61462 Königstein (DE)
(86) Internationale Anmeldenummer: PCT/EP2000/005586
(87) Internationale Veröffentlichungsnummer: WO 2001/005982

(56) Entgegenhaltungen:
- EP-A- 0 358 428
- WO-A-97/41214
- WO-A-98/27201
- DE-A- 4 308 061
- GB-A- 2 031 896

## Beschreibung

Die Erzeugung der nicht-proteinogenen Aminosäure L-PPT (L-Phosphinothricin) durch Racemat-Trennung ist bisher mit hoher Reinheit und Ausbeute nur durch Spaltung von Phenacetyl-Phosphinothricin mit Penicillin-G-Acylase aus *Escherichia coli* beschrieben worden (DE-A-3048612). Die Synthese von Phenacetyl-PPT ist aber im Vergleich zu N-Ac-PPT aufwendiger und teurer. Jedoch besitzt Penicillin-G-Acylase keine Spezifität für aliphatische Acylreste und somit auch nicht für N-Ac-PPT. Andere bekannte Acylasen haben ebenfalls keine oder nur geringe Substratspezifität für N-Ac-PPT und wurden bisher nur in mikrobiellen Biotransformationen ohne Aufreinigung der Enzyme eingesetzt (wie z.B. in DE-A-2939269 beschrieben). Dadurch waren nur sehr geringe Raum/Zeit-Ausbeuten erreichbar. Aus der Patentanmeldung EP-A-0382113 ist die L-spezifische Spaltung von N-Ac-PPT Carbonsäureestem durch Acylase I bekannt. Auch dieses Enzym hat jedoch keine Spezifität für die freie Carbonsäure und erfordert deshalb eine Veresterung als zusätzlichen Syntheseschritt bei der Substratbereitstellung. EP 0358428 beschreibt die Konvenion ausschliesslich des 2-Isomer von N-substituierter Carbonyl-DL-Aminosäuren durch stereoselektive Eliminierung der substituierten Carbonylgruppe mittels Mikroorganismen, die eine Acylase-Aktivität besitzen, wobei die Reaktionen mit geringen Raum/Zeit-Ausbeuten ablaufen.

Die Patentanmeldung WO 98/27201 beschreibt die gezielte Isolierung von mikrobiellen Deacetylasen aus Bodenproben mit Spezifität für N-Acetyl-Aminosäuren, vorzugsweise N-Acetyl-Phosphinathricin (N-Ac-PPT), sowie die Klonierung der korrespondierenden Gene aus *Stenotrophomonas sp.* und Comamonas acidovorans.
Aufgrund der gefundenen Sequenzhomologien und der durchgeführten Substratspezifitätstests konnte gezeigt werden, daß die beschriebenen Deacetylasen zur Gruppe der Hippurat-Hydrolasen (EC 3.5.1.32) gehören, deren natürliches Substrat N-Benzoyl-Glycin, ein Aminosäure-Derivat mit einer aromatischen N-Acylfunktion ist. Daher war es interessant, daß diese Enzyme auch N-acetylierte Aminosäuren, insbesondere N-Acetyl-Phosphinothdcin, als Substrat akzeptieren können, wobei es sich um Aminosäure-Derivate mit aßhatischen N-Acylfunktionen handelt. Aus den Patentanmeldungen GB 2031896 und DE-A-2717440 ist bekannt, daß die herbizide Wirkung von racemischem Phosphinothricin allein von dessen L-Enantiomer (L-PPT) ausgeht.
In diesem Zusammenhang war es interessant, daß die gefundenen Deacetylasen ausschließlich die L-Enantiomere von N-Acetyl-PPT, sowie von N-Acetyl-Derivaten einiger proteinogener Aminosäuren mit hoher Spezifität spalten. Daher eignen sich diese Enzyme ausgezeichnet zur Herstellung von L-Aminosäuren, insbesondere des herbiziden Wirkstoffs L-Phosphinothricin, aus deren racemischen N-Acetyl-Derivaten nach dem Prinzip der Racemat-Spaltung, wie es z.B. in den Patentanmeldungen EP-A-0304021, GB 2031896 und DE-A-3048612 beschrieben wird. Wesentliche Nachteile der Verfahren in den drei vorangehend beschriebenen Patentanmeldungen bestehen jedoch darin, daß (1) nur geringe Substratkonzentrationen (ca. 0.5% im Falle der Patentanmeldung DE-A-2939269) eingesetzt werden können, wodurch eine technische Eignung als gering zu bewerten ist, daß (2) mit nicht isolierten Enzymen gearbeitet wird, wodurch sich die Problematik von Nebenreaktionen und anschließender Aufreinigungsschritte nicht oder nur sehr kostenintensiv lösen läßt und daß (3) eine kostenintensive Produktbereitstellung notwendig ist (wie im Falle der Patentanmeldung DE-A-3048612).

Die der Erfindung zugrunde liegende Aufgabe liegt darin, eine Deacetylase (wie in WO 83/27201 bereits charakterisiert) in geeigneter Form und Menge zu exprimieren, und es mit Hilfe dieses Enzyms zu ermöglichen, L-PPT demchemisch sehr einfach zugänglichen racemischen N-Acetyl-D,L-PPT durch Racemat-Spaltung mit hoher Ausbeute und Enantiomerenreinheit herzustellen.

Für den Fall, daß die enzymatische Aktivität bereits annähernd bekannt ist, und in dem auch bereits eine Klonierung der das Enzym codierenden Nukleinsäuresequenz erfolgt ist, besteht die erste Tätigkeit darin, das entsprechende Nukleinsäurefragment in einen geeigneten Vektor zu überführen, um dann entweder eine Überproduktion in einem geeigneten Bakterienstamm durchzuführen, und/oder um das Protein nach der Überexpression zu isolieren. Das so isolierte Enzym kann entweder direkt zur enzymatischen Reaktion verwendet werden oder aber über geeignete Kopptungsgruppen an eine Matrix fixiert werden.

Eine erster enzymatischer Test ist empfehlenswert, um die generelle Bestätigung zu erhalten, ob die gewünschte Reaktion abläuft. Alle weiteren Schritte dienen der Parameteroptimierung hinsichtlich der Reaktionsspezifität (bezogen auf Edukt und Produkt), der Reaktionsgeschwindigkeit, der Reaktionseffizienz, der Halbwertszeit des eingesetzten Enzyms, sowie der möglichen Substratkonzentrationen. Für den Fall, daß einzelne Parameter nicht genau den Anforderungen entsprechen, ist man in der Lage, geeignete Veränderungen an der das Enzym kodierenden Nukleinsäuresequenz vorzunehmen, die eine weitere Optimierung des vorhandenen natürlichen Enzyms zur Folge haben.

Die Klonierung der verwendeten Deacetylase wurde bereits in der Anmeldung WO 98/27201 beschrieben. Das für die Deacetylasen deac1 kodierende Nukleinsäurefragment wurde wie im Beispiel 1 weiter unten ausgeführt in die entsprechenenden Expressionsvektoren umkloniert, um somit ein notwendiges Maß an eindeutiger Substratspezifität und die Abwesenheit von Nebenreaktionen sicherzustellen.

Die Erfindung betrifft ein Verfahren zur Herstellung von L-PPT aus racemischem N-Acetyl-D,L-PPT, dadurch gekennzeichnet, daß
(a) durch eine enzymatische Racemat-Spaltung mittels der isolierten rekombinantenHippurat-Hydrolase deac1 selektiv N-Acetyl-L-PPT deacetyliert wird, während N-Acetyt-D-PPT wird nicht deacetyliert wird,
(b) das racemische N-Acetyl-D,L-PPT in einer Konzentration von größer 50 mM bis 1500 mM vorliegt,
(c) das erhaltene deacetylierte L-PPT präparativ von dem nicht deacetylierte N-Acetyl-D-PPT und/oder dem nicht vollständig deacetylierten N-Acetyl-L-PPT getrennt wird, und wobei
(d) die Raum-Zeit-Ausbeute an erhaltenem deactylierten L-PPT bei einer Reaktionszeit von 55 Stunden bei mindestens 69 mg deaceyliertern-L-PPT/g Biokatalysator/h liegt.

Die Erfindung betrifft insbesondere ein Verfahren zur Herstellung von L-Phosphinothricin (L-PPT) aus N-Acetyl-D,L-Phosphinothricin durch enzymatische Racemat-Spaltung mittels isolierter, rekombinanter Enzyme.

Des weiteren betrifft die Erfindung die Verwendung der erzeugten rekombinanten deac1 als Biokatalysatore, die es erlaubt, die entsprechenden Reaktionen mit hohen Substratkonzentrationen und/oder der Erzielung hoher Raum/Zeit-Ausbeuten durchzuführen, insbesondere unter Verwendung der rekombinanten deac1 in immobilisierter Form.

Weiterhin betrifft die Erfindung die Durchführung der oben beschriebenen Verfahren bei Reaktionstemperaturen von etwa 25°C bis 65°C, bevorzugt bei Reaktionstemperaturen von etwa 30°C bis 45°C und besonders bevorzugt bei Reaktionstemperaturen von etwa 35°C bis 40°C.

Des weiteren betrifft die Erfindung die Durchführung der oben beschriebenen Verfahren bei Substratkonzentrationen von etwa 10 mM bis 1500 mM, bevorzugt bei Substratkonzentrationen größer als 50 mM, besonders bevorzugt bei Substratkonzentrationen größer als 250 mM und ganz besonders bevorzugt bei Substratkonzentrationen größer als 500 mM.

Weiterhin betrifft die Erfinfung die Trennung des mit Hilfe der oben beschriebenen Schritte erzeugten L-PPT von N-Acetyl-D-PPT und nicht vollständig umgesetzter N-Acetyl-L-PPT.

Nutzbare Verfahren sind hierbei die Verwendung der Ionenaustauscherchromatographie an einem sauren lonenaustauscher oder aber die Extraktion der N-Acetyl-D,L-PPT mittels eines organischen Lösungsmittels, wie beispielsweise von Methylisobutylketon, wobei das vorangehend erzeugte L-PPT in die wäßrige Phase übergeht, aus der es dann durch Trocknung aufkonzentriert wird.

### Beispiele:

### 1. Produktion des deac-Enzyms als rekombinantes Protein in Escherichia coli:

Das *deac* 1-Strukturgen aus *Stenotrophomonas sp.,* codierend für eine N-Ac-PPTspezifische Deacetylase, wurde als 1,4-kb BamHI/SalI Fragment (beschrieben in WO 98/27201) in die *Bam* HI/*Sal* I-Schnittstelle des HisTag-Expressionsvektor pQE30 der Firma Quiagen kloniert [Quiaexpress Kit, Type IV, Katalog Nr. 32149, Stüber et al. (1990), in: Immunological Methods, Lefkovits, I. and Pemis, B., eds., vol. IV, Academic Press, New York, pp. 121-152].
Alle molekularbiologischen Arbeiten wurden nach Standardprotokollen durchgeführt, wie z.B. bei Ausubel et al. (1995), Current protocols in molecular biology, John Wiley and Sons, New York, beschrieben. Das religierte Konstrukt wurde in den vom Hersteller (Qiagen) empfohlenen Bakteriestamm *E. coli* M15 transformiert, und rekombinante Klone wurden auf 100 µg/ml Ampicillin und 25 µg/ml Kanamycin selektiert. Die Expression von rekombinantern Fusionsprotein wurde durch Zugabe von 1 mM IPTG induziert. Die Zellen wurden 4-5 h nach Induktion abgeemtet und bis zur Aufarbeitung des Proteins bei -20°C aufbewahrt.
Durch SDS/Polyacrylamid-Elektrophorese von Zellaliquots der induzierten Klone konnte das HisTag-*deac*1-Fusionsprotein als zusätzliche Bande von 49-kDa nachgewiesen werden.
Der Nachweis der enzymatischen Aktivität des Deacetylase-Fusionsproteins erfolgte im radioaktiven Assay mit [¹⁴C]-N-Acetyl-L-PPT als Substrat. Dazu wurden je 200 µl der induzierten Kulturen mit 0,5 % Toluol, 0,5 % Ethanol für 30 min. bei 37°C permeabilisiert. Die Zellpellets wurden anschließend in je 25 µl 0,1 mM [¹⁴C]-N-Acetyl-L-PPT, 10 mM NaCl, 10 mM NaPhosphat, pH = 7,5 resuspendiert und für 1 h bei 37°C inkubiert. Zur qualitativen Bestimmung des gebildeten [¹⁴C]-L-PPT wurden je 5 µl Aliquots der Testansätze durch Dünnschichtchromatographie auf HPTLC-Zellulose-Platten (Merck) mit n-Propanol : 25 % Ammoniak = 3 : 2 als Laufmittel analysiert. Die radioaktiven Banden wurden durch Autoradiographie auf Röntgenfilm sichtbar gemacht.
Zur Quantifizierung der Enzymumsetzung wurden die Testansätze in der Radio-HPLC mit Spherisorb® SAX als Trennsäule gemessen (Laufmittel: 5 mM KH₂PO₄, 10 % Methanol, pH = 1,92, Flußrate: 0,5 ml/min.). Unter diesen Bedingungen eluiert [¹⁴C]-L-PPT bei 4,5 min. und [¹⁴C]-N-Acetyl-L-PPT bei 6,5 min.
Der Expressionsklon mit der höchsten N-Acetyl-L-PPT-spezifischen Deacetylase-Aktivität produziert das Deacetylase-Protein gemäß einer semiquantitativen Bestimmung mit einem Anteil von ca. 10 % am Gesamtprotein und wurde für die Enzymreinigung und die nachfolgend beschriebenen Biotransformationen verwendet.

### 2. Reinigung der Deacetylase durch Affinitätschromatographie:

Die Isolierung des Deacetylase-Proteins aus dem nach der im Beispiel 1 beschriebenen Klonierung neu benannten Expressionsstamm pQEDEAC erfolgte nach Quiagen Protokoll (Qiaexpress-Kit) durch Affinitätschromatographie an Nickel-Nitrilotriacetat-Matrix (Ni-NTA) unter nativen Bedingungen. Dazu wurden 800 ml Kultur des Expressionsstammes pQEDEAC wie in Beispiel 1 beschrieben fermentiert und induziert. Das geerntete Zellpellet (4 g) wurde in 20 ml Lysispuffer resuspendiert und mit Ultraschall aufgeschlossen. Nach Abzentrifugieren wurde das klare Proteinlysat (120 mg Gesamtprotein) mit 4 ml 50 % iger Ni-NTA-Suspension zur Bindung des HisTag-Fusionsproteins versetzt und das Material anschließend auf eine Enzymsäule geladen. Die Affinitätsmatrix wurde mit 10 Volumen Waschpuffer gewaschen und dann mit 4 ml Elutionspuffer eluiert. Zur Überprüfung der Affinitätsreinigung wurden Aliquots des Zellaufschlusses, der Waschlösungen und des Eluats mittels SDS/Polyacrylamid-Gelelektrophorese analysiert. Die Deacetylase-Aktivität der verschiedenen proteinhaltigen Fraktionen wurde mit den in Beispiel 1 beschriebenen radioaktiven Assays bestimmt (Reaktionsansätze: 9 µl Proteinlösung + 1 µl 1 mM [¹⁴C]-N-Acetyl-L-PPT, Reaktionsbedingungen: siehe oben). Zur quantitativen Enzymbestimmung wurden die Proteinfraktionen mit 50 mM N-Acetyl-D,L-PPT (9 µl Proteinlösung + 1 µl 500 mM N-Acetyl-D,L-PPT) für 30 min. bei 37°C inkubiert. Das entstandene PPT wurde anschließend im Aminosäureanalysator (Biotronic LC 5001) gemessen.
Das Protein im Rohextrakt nach Zellaufschluß hatte eine spezifische Deacetylase-Aktivität von 3 nkat/mg Protein (1 nkat = 1 nmol Substrat/sec.). Aus 800 ml Kultur des Expressionsstammes konnten durch Affinitätschromatographie 10 mg HisTag*deac*-Fusionsprotein mit einer spezifischen Aktivität von 20 nkat/mg Protein und einer Reinheit von ca. 80 % isoliert werden.

### 3. Substratspezifität und Stereoselektivität der Deacetylase:

Zur Untersuchung von Substrat- und Stereospezifität des *deac*1-Proteins wurden je 5 µg gereinigtes Enzym in 20 µl Ansätzen in 10 mM NaCl, 10 mM NaPhosphat, pH = 7,5 mit je 25 mM der in Tabelle 1 genannten N-Acetyl-L-Aminosäuren bzw. der entsprechenden D-Enantiomere inkubiert. Zum Vergleich wurde Hippursäure (N-Benzoyl-Glycin), das natürliche Substrat der Deacetylase, eingesetzt. Die Reaktionen wurden für 1 h bei 37°C inkubiert und anschließend auf Bildung der freien Aminosäuren im Aminosäureanalysator gemessen (Biotronic LC 5001). Tabelle 1 gibt die relativen Deacetylase-Aktivitäten für die L-Enantiomere der verschiedenen Substrate an. Neben Hippursäure und N-Ac-L-PPT spaltet das *deac*1-Enzym auch noch die N-Acetyl-Derivate einer Reihe natürlicher Aminosäuren, insbesondere N-Ac-L-Glutaminsäure, N-Ac-Glycin, N-Ac-L-Histidin, N-Ac-L-Omithin, N-Ac-L-Leucin, N-Ac-L-Glutamin und N-Ac-L-Phenylalanin. In allen Fällen war das Enzym ausschließlich mit den L-Enantiomeren aktiv, während die entsprechenden D-Verbindungen in keinem Fall umgesetzt werden konnten. In allen anderen getesteten Fällen (siehe Tabelle 1) fand die Umsetzung der N-Acetyl-L-Derivate nur in einem sehr geringen Maß oder aber überhaupt nicht statt.

**Tabelle 1: Substratspezifität des deac1-Enzyms**

| Substrat¹ | Rel. Aktivität² [%] |
|---|---|
| Hippursäure (N-Benzoyl-Glycin) | 100 |
| N-Ac-L-Phosphinothricin | 43 |
| N-Ac-L-Omithin | 37 |
| N-Ac-L-Methionin | 0 |
| N-Ac-L-Tryptophan | 0,4 |
| N-Ac-L-Phenylalanin | 24 |
| N-Ac-L-Tyrosin | 11 |
| N-Ac-L-Glutaminsäure | 100 |
| N-Ac-L-Glutamin | 30 |
| N-Ac-Glycin | 59 |
| N-Ac-L-Histidin | 43 |
| N-Ac-L-Leucin | 33 |
| N-Ac-L-Valin | 2 |
| N-Ac-L-Serin | 4 |
| N-Ac-L-Prolin | 0 |

| | |
|---|---|
| ¹: Bei allen Verbindungen handelt es sich um die L-Enantiomere | |
| ²: Die mit Hippursäure gemessene spezifische Aktivität [nmol Glycin/min./mg Protein] wurde gleich 100 % gesetzt, und die anderen Werte wurden darauf bezogen | |

### 4. Racemat-Spaltung von N-Acetyl-D,L-Phosphinothricin durch Biotransformation mit Zellen des deac1-Produzentenstammes:

Eine 400 ml Kultur des Expressionsstammes pQEDEAC wurde, wie in Beispiel 1 beschrieben, fermentiert und induziert. Die geemteten Zellen wurden anschließend in 10 mM NaCl, 10 mM NaPhosphat, pH = 7,5 gewaschen und über Nacht lyophilisiert. Die so behandelten Zellen konnten über mehrere Wochen bei 4°C unter Beibehaltung einer hohen spezifischen Deacetylase-Aktivität gelagert werden und dienten in den folgenden Versuchen als Biokatalysator.
2 mg der lyophilisierten Zellen wurden in 1 ml der folgenden Substratlösungen variierender Konzentrationen resuspendiert:
(1) 10 mM N-Acetyl-D,L-PPT, Dinatrium-Salz (0,3 %)
(2) 50 mM " " (1,3 %)
(3) 100 mM " " (2,6 %)
(4) 250 mM " " (6,7 %)
(5) 500 mM " " (13,3 %)

Die Ansätze enthielten als Reaktionspuffer 10 mM NaCl, 10 mM NaPhosphat, pH = 8,0 und wurden für 48 h auf dem Schüttler bei 100 rpm und 37°C inkubiert. Das bei der Hydrolyse-Reaktion gebildete freie PPT wurde im Aminosäureanalysator (Biotronic LC 5001) bestimmt. Die Ergebnisse sind in Tabelle 2 zusammengefaßt. Die Enantiomerenreinheit des Reaktionsproduktes wurde durch chirale HPLC mit der Trennsäule Chirex® (D) Penicillamin (Phenomenex) analysiert. Als Laufmittel wurde 2 mM CuSO₄, 10 % Methanol mit einer Flußrate von 0,5 ml/min. verwendet. Die Detektion erfolgt photometrisch bei 254 nm. Unter diesen Bedingungen eluieren Referenzlösungen von L-PPT bei 17 min. und D-PPT bei 21 min. In allen Testproben konnte ausschließlich L-PPT als Reaktionsprodukt nachgewiesen werden. Die erzielten Konversionsraten [produziertes L-PPT/N-Acetyl-L-PPT im Substrat x 100] lagen zwischen 100 % bei den niedrigen Substratkonzentrationen (10 mM, 50 mM) und 66 % bei der höchsten verwendeten Substratkonzentration (500 mM).

**Tabelle 2: Racemat-Spaltung von N-Acetyl-D,L-PPT in Abhängigkeit von der Substratkonzentration**

| Substratlösung N-Acetyl-D,L-PPT [mM] | Produktlösung* L-PPT [mM] | Konversionsrate L-PPT/N-Acetyl-D,L-PPT in % |
|---|---|---|
| 10 | 5 | 100 |
| 50 | 25 | 100 |
| 100 | 48 | 96 |
| 250 | 108 | 86 |
| 500 | 164 | 66 |

| | | |
|---|---|---|
| *: Konzentration an Biokatalysator: 2 mg/ml | | |

Im folgenden Versuch wurde die Konzentration an Biokatalysator erhöht und eine Reaktionskinetik der Substratspaltung aufgenommen. 13 mg der lyophilisierten Zellen wurden in 1 ml Substratlösung, bestehend aus 500 mM N-Acetyl-D,L-PPT, Dinatrium Salz (13,3 % w|v), 10 mM NaCl, 10 mM NaPhosphat, pH = 8,0 resuspendiert und auf einem Schüttler bei 100 rpm und 37°C inkubiert. Zur Bestimmung des gebildeten L-PPT wurden über einen Zeitraum von 55 h je 50 µl Aliquots aus dem Reaktionsansatz abgenommen, zentrifugiert und die Überstände bis zur Analyse bei -20°C eingefroren. Die quantitative Analyse des Reaktionsproduktes L-PPT, sowie die Bestimmung der Enantiomerenreinheit wurden wie oben beschrieben durchgeführt. Die Ergebnisse sind in Tabelle 3 zusammengefaßt. Unter den gewählten Bedingungen war die L-spezifische SubstratSpaltung nach ca. 55 h abgeschlossen. Dabei wurden eine Konversionsrate von ca. 90 % erreicht.

**Tabelle 3: Reaktionskinetik der Racemat-Spaltung von N-Acetyl-D,L-PPT**

| Reaktionszeit [h] | L-PPT [mM]* |
|---|---|
| 0 | 0 |
| 1 | 27,7 |
| 4 | 59,7 |
| 9 | 106,0 |
| 23 | 181,6 |
| 31 | 186,5 |
| 47 | 208,6 |
| 55 | 221,8 |

| | |
|---|---|
| *: Konzentration an Biokatalysator: 13 mg/ml Substratkonzentration: 500 mM N-Acetyl-D,L-PPT | |

### 5. Immobilisierung des gereinigten deac1-Proteins:

Um die Enzymreaktion mit dem isolierten *deac*1-Protein durchführen zu können, wurde das in Beispiel 2 als HisTag-Fusionsprotein gereinigte Enzym an den Polymer-Träger VINA-Epoxy Biosynth® (Riedel de Haen) immobilisiert. Dazu wurde das *deac*-Protein durch Ammoniumsulfat-Fällung auf 15 mg/ml aufkonzentriert und über Nacht gegen 1 M NaPhosphat, pH = 8,0 dialysiert. Für eine Standard-Kopplungsreaktion wurden 10 mg des Proteins mit 100 mg VINA-Träger für 2 Tage bei Raumtemperatur langsam geschüttelt. Anschließend wurde das Immobilisat abzentrifugiert und je 1x in Immobilisierungspuffer, sowie in 50 mM NaPhosphat, pH = 7,0 gewaschen. Zum Blocken freier Epoxy-Gruppen wurde der Träger dann für 1 h bei Raumtemperatur mit 50 mM NaPhosphat, pH = 7,0, 50 mM 2-Mercaptoethanol inkubiert. Der Biokatalysator wurde danach in 1ml 5 mM NaPhosphat, pH = 7,0, 0,02 % NaAzid bei 4°C gelagert. Zur Bestimmung der Proteinkopplung wurde der Proteingehalt der Lösung vor und nach Immobilisierung, sowie in den Waschlösungen mit Hilfe der Bradford Methode bestimmt [Bradford (1976), Anal. Biochem. 72: 248-254]. Die spezifische Aktivität der Deacetylase wurde vor und nach Immobilisierung mit dem in Beispiel 1 beschriebenen radioaktiven Assay gemessen. Die relative Enzymaktivität war nach Kopplung > 90 % des für das gelöste Protein bestimmten Wertes.

### 6. Racemat-Spaltung von N-Acetyl-D,L-Phosphinothricin mit immobilisiertem deac1-Enzym im Säulenreaktor:

I g Enzym-Immobilisat (Feuchtgewicht) wurde in einen Säulenreaktor geladen und als Biokatalysator für die Racemat-Spaltung von verschieden konzentrierten Substratlösungen mit verschiedenen Flußraten benutzt. Die Substratlösungen enthielten die in Tabelle 4 angegebenen Konzentrationen an N-Acetyl-D,L-PPT, Diammoniumsalz in 10 mM NaCl, 10 mM NaPhosphat, pH = 8,0. Die Reaktionen wurden bei 37°C über insgesamt 10 Tage durchgeführt. Die Umsetzungen wurden durch Bestimmung des im Säulendurchfluß vorhandenen L-PPT mittels Aminosäureanalyse und chiraler HPLC quantifiziert (siehe Beispiel 4). Die Ergebnisse sind in Tabelle 4 dargestellt. Die höchste Konversionsrate von 83 % wurde mit der höchsten Substratkonzentration (500 mM) und dem niedrigsten Substratfluß (0,03 ml/min.) erreicht. Die Raum/Zeit-Ausbeute stieg mit der Flußrate und der Substratkonzentration an und erreichte den höchsten Wert von 181 [mg L-PPT/g Biokatalysator/h] bei einer Substratkonzentration von 500 mM und einem Fluß von 0,3 ml/min. Die immobilisierte Deacetylase zeigte über den gesamten Versuchszeitraum eine gute Stabilität und besaß nach 10 Tagen bei 37°C noch ca. 80 % der Ausgangsaktivität.

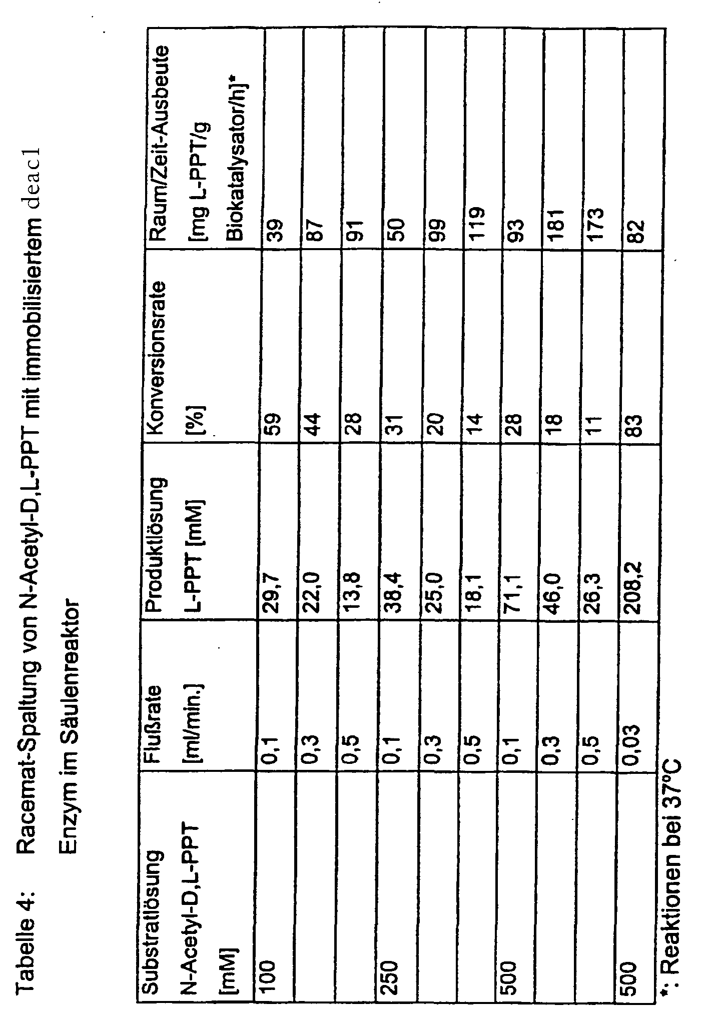

## Patentansprüche

1. Verfahren zur Herstellung von L-PPT aus dem racemischen N-Acetyl-D,L-PPT, **dadurch gekennzeichnet, daß**
(a) durch eine enzymatische Racemat-Spaltung mittels der isolierten rekombinanten Hippurat-Hydrolase deac1 aus Stenotrophomonas sp. selektiv N-Acetyl-L-PPT deacetyliert wird, während N-Acetyl-D-PPT nicht deacetyliert wird,
(b) das racemische N-Acetyl-D,L-PPT in einer Konzentration von größer 50 mM bis 1500 mM vorliegt,
(c) das erhaltene deacetylierte L-PPT präparativ von dem nicht deacetylierten N-Acetyl-D-PPT und/oder dem nicht vollständig deacetylierten N-Acetyl-L-PPT getrennt wird, und wobei
(d) die Raum-Zeit-Ausbeute an erhaltenem deactylierten L-PPT bei einer Reaktionszeit von 55 Stunden bei mindestens 69 mg deaceyliertern L-PPT/g Biokatalysator/h liegt.

2. Verfahren gemäß Anspruch 1, wobei das rekombinante deac1 in immobilisierter Form eingesetzt wird.

3. Verfahren gemäß Anspruch 1 oder 2, wobei die Reaktionstemperaturen etwa 25°C bis 65°C betragen.

4. Verwendung von deac1 in einem Verfahren gemäß einem oder mehreren der Ansprüche 1 bis 3 zur enantioselektiven Erzeugung von L-PPT aus dem racemischen N-Acetyl-D,L-PPT.

## Claims

1. A process for the preparation of L-PPT from racemic N-acetyl-D,L-PPT, **characterized in that**
(a) N-acetyl-L-PPT is deacetylated, while N-acetyl-D-PPT is not deacetylated, by an enzymatic racemate resolution by means of the isolated recombinant hippurate hydrolase deac1 from Stenotrophomonas sp.,
(b) the racemic N-acetyl-D,L-PPT is present in a concentration of greater than 50 mM to 1500 mM,
(c) the deacetylated L-PPT obtained is preparatively separated from the nondeacetylated N-acetyl-D-PPT and/or from the incompletely deacetylated N-acetyl-L-PPT, and wherein
(d) the space-time yield of deacetylated L-PPT obtained in the course of a reaction time of 55 hours is at least 69 mg of deacetylated L-PPT/g of biocatalyst/h.

2. The process as claimed in claim 1, wherein the recombinant deac1 is employed in immobilized form.

3. The process as claimed in claim 1 or 2, wherein the reaction temperatures are approximately 25°C to 65°C

4. The use of deac1 in a process as claimed in one or more of claims 1 to 3 for the enantioselective production of L-PPT from racemic N-acetyl-D,L PPT.

## Revendications

1. Procédé pour la préparation de L-PPT à partir du N-acétyl-D,L-PPT racémique, **caractérisé en ce que**
(a) le N-acétyl-L-PPT est désacétylé sélectivement par un dédoublement racémique enzymatique à l'aide de l'hippurate hydrolase recombinante deac 1, isolée à partir de *Stenotrophomonas sp.,* alors que le N-acétyl-D-PPT n'est pas désacétylé,
b) le N-acétyl-D,L-PPT racémique est présent en une concentration supérieure à de 50 mmoles à 1500 mmoles,
c) le L-PPT désacétylé obtenu est séparé par préparation du N-acétyl-D-PPT non désacétylé et/ou du N-acétyl-L-PPT non complètement désacétylé,
et
d) le rendement espace-temps en L-PPT désacétylé est, pour un temps de réaction de 55 heures, d'au moins 69 mg de L-PPT désacétylé/g de biocatalyseur/heure.

2. Procédé selon la revendication 1, la deac 1 recombinante étant utilisée sous forme immobilisée.

3. Procédé selon la revendication 1 ou 2, les températures réactionnelles étant d'environ 25°C à 65°C.

4. Utilisation de la deac 1 dans un procédé selon une ou plusieurs des revendications 1 à 3 pour la production énantiosélective de L-PPT à partir du N-acétyl-D,L-PPT racémique.
